# EUROPEAN PATENT APPLICATION

(11) **EP 3 984 585 A1**
(43) Date of publication of application: **20.04.2022**
(21) Application number: 20822949.2
(22) Date of filing: 03.06.2020
(51) Int. Cl.: A61M 37/00

(54) **MICRONEEDLE ARRAY UNIT AND CONTAINER**

(30) Priority: 11.06.2019 JP 2019108832
(71) Applicant: FUJIFILM Corporation, Tokyo 106-8620 (JP)
(72) Inventor: YOSHIDA, Junya, Ashigarakami-gun, Kanagawa 258-8577 (JP); YONEYAMA, Satoshi, Ashigarakami-gun, Kanagawa 258-8577 (JP)
(74) Representative: Klunker IP Patentanwälte PartG mbB
(86) International application number: PCT/JP2020/021931
(87) International publication number: WO 2020/250770

(57) **Abstract**

An object of the present invention is to provide a micro-needle array unit and a container, to which gas permeability, internal visibility, and sterility are imparted. A micro-needle array unit (1) includes a micro-needle array (40) and a container (10) which accommodates the micro-needle array (40), in which the container (10) includes an accommodating portion (12) having an opening (12A), a claw portion (54) provided in the accommodating portion (12) and supporting an outer peripheral surface (42A) of one surface (42) of the micro-needle array (40), a deformable portion (14) disposed on a side opposite to the opening (12A), a flange portion (16) formed integrally with the accommodating portion (12), and a lid member (30) provided in close contact with the flange portion (16), at least a part of the lid member (30) is formed of a transparent film (32), and a water vapor permeable antibacterial film (34) is provided on at least a part of the container (10).

## Description

### BACKGROUND OF THE INVENTION

### 1. Field of the Invention

The present invention relates to a micro-needle array unit and a container.

### 2. Description of the Related Art

In the related art, as methods for administering a drug or the like to the surface of a living body, that is, the skin or the mucous membrane, methods of attaching a liquid substance or a powdery substance to the surface thereof have been used in most cases. However, since the region where the substance is attached is limited to the surface of the skin, the attached drug or the like is removed due to perspiration or contact of foreign matter, and thus administration of an appropriate amount of the drug or the like is difficult to perform. Further, in a case where a method using permeation of such a drug into the skin through diffusion is employed, the drug is blocked by a horny barrier layer, and thus sufficient drug efficacy is unlikely to be obtained. Particularly in biopharmaceuticals, which have been attracting attention in recent years, administration by injection has been selected due to extreme difficulty in breaking through the barrier layer using permeation.

However, administration by injection requires hands of healthcare professionals and is associated with pain and risk of infection. Under the above-described circumstances, a method of allowing micro-needles to penetrate through a horny barrier layer to inject a drug into the skin without pain using a micro-needle array in which micro-needles containing a drug and having a high aspect ratio (also referred to as "needles" or "needle-like convex portions") are formed has been attracting attention.

Since the micro-needle array is used by puncturing the skin, it is necessary to protect micro-needles until the micro-needles puncture the skin. Further, in order to ensure the sterility of the micro-needles, it is preferable that the micro-needles are stored in a container until immediately before use.

For example, JP2018-191783A describes, as such a micro-needle unit consisting of a micro-needle array and a container, a micro-needle array unit including protruding portions in an accommodating portion of a container, in which the micro-needle array is supported by the protruding portions and a deformable portion on a side opposite to an opening of the container is pressed so that micro-needles are pushed out of the accommodating portion. WO2015/005143A describes that micro-needles are stored in a concave container and the bottom portion of the container is pressed toward the opening of the container so that the micro-needles penetrate through a liquid support and the tips of the micro-needles puncture the skin.

### SUMMARY OF THE INVENTION

A micro-needle array has been attracting attention in recent years as an alternative to injection administration. Therefore, it is preferable that a packaging container thereof is provided with a function of maintaining a sterile state and a function of visually recognizing the inside in order to inspect foreign matter in a pharmaceutical preparation, similar to a case of injection.

Further, in order to improve the stability of the drug in the micro-needle array, it is necessary to dry the micro-needle array until the micro-needle array enters a low water content state, but in order to ensure a sterile state in the container, it is necessary to dry the micro-needle array before packaging using equipment that dries the micro-needle array until the micro-needle array enters a low water content state in an expensive sterile environment.

In the micro-needle array unit described in JP2018-191782A and the micro-needle unit described in WO2015/005143A, the micro-needle array needs to be dried until the micro-needle array enters a low water content state in a sterile environment, and thus the drying step is a costly step in a step of producing the micro-needle array. Further, the visibility of the micro-needle array inside the container is also not described in JP2018-191782A and WO2015/005143A.

The present invention has been made in consideration of the above-described circumstances, and an object thereof is to provide a micro-needle array unit including a micro-needle array and a container to which gas permeability, internal visibility, and sterility are imparted and the container.

In order to achieve the object of the present invention, there is provided a micro-needle array unit comprising: a micro-needle array which includes a sheet and a plurality of needles arranged inside an outer peripheral surface of one surface of the sheet; and a container which accommodates the micro-needle array, in which the container includes an accommodating portion having an opening, a claw portion provided in the accommodating portion and supporting the outer peripheral surface of the one surface of the micro-needle array, a deformable portion disposed on a side opposite to the opening and formed integrally with the accommodating portion, a flange portion extending from a periphery of the opening and formed integrally with the accommodating portion, the flange portion consisting of an outer flange portion that comes into contact with a skin in a case where the skin is punctured by the micro-needle array and an inner flange portion that is provided inside the outer flange portion, and a lid member provided in close contact with the inner flange portion, at least a part of the lid member is formed of a transparent film, a water vapor permeable antibacterial film is provided on at least a part of the container, the deformable portion is deformed by receiving an external force in a direction of the opening and presses the other surface of the micro-needle array, the micro-needle array is pushed out of the accommodating portion by pressing the other surface, and the deformable portion maintains a deformed state and presses the micro-needle array.

In order to achieve the object of the present invention, there is provided a container which accommodates a micro-needle array having a sheet and a plurality of needles arranged inside an outer peripheral surface of one surface of the sheet, the container comprising: an accommodating portion which has an opening; a claw portion which is provided in the accommodating portion and supports the outer peripheral surface of the one surface of the micro-needle array; a deformable portion which is disposed on a side opposite to the opening and formed integrally with the accommodating portion; a flange portion which extends from a periphery of the opening and is formed integrally with the accommodating portion, the flange portion consisting of an outer flange portion that comes into contact with a skin and an inner flange portion that is provided inside the outer flange portion; and a lid member which is provided in close contact with the inner flange portion, in which at least a part of the lid member is formed of a transparent film, a water vapor permeable antibacterial film is provided on at least a part of the container, the deformable portion is deformed by receiving an external force in a direction of the opening and presses the other surface of the micro-needle array, the deformable portion maintains a deformed state and presses the micro-needles.

According to the micro-needle array unit of the present invention, the micro-needle array can be dried in a state of being stored in the container by using a part of the container as a film having water vapor permeability and sterility (hereinafter, also referred to as a water vapor permeable antibacterial film). Further, since the film is an antibacterial film, invasion of bacteria into the container can be prevented, and the sterile state inside the container can be maintained. In this manner, since the micro-needle array can be dried in a sterile state, the micro-needle array is not required to be dried in an expensive sterile room for a long time. Therefore, the production cost of the micro-needle array can be reduced. Further, since the transparent film is used as the lid member, the visibility inside the container can be ensured, and thus foreign matter inspection and shape inspection can be performed on the micro-needles in a packaged state. Therefore, it is not necessary to perform these inspections in an expensive sterile room, and thus the production cost can be reduced.

As described above, according to the micro-needle array unit of the present invention, it is possible to ensure the sterility, the gas permeability, and the internal visibility.

### BRIEF DESCRIPTION OF THE DRAWINGS

Fig. 1 is a perspective view illustrating a micro-needle array unit.
Fig. 2 is a cross-sectional view of the micro-needle array unit illustrated in Fig. 1.
Fig. 3 is a plan view of the micro-needle array unit illustrated in Fig. 1.
Fig. 4 is a perspective view illustrating a micro-needle array.
Fig. 5 is a perspective view illustrating a fixing member.
Fig. 6 is a view illustrating a state in which the fixing member is fitted to a resin block.
Fig. 7 is a view for describing a step of puncturing the skin with the micro-needle array.
Fig. 8 is a view for describing a step of puncturing the skin with the micro-needle array.
Fig. 9 is a view for describing a step of puncturing the skin with the micro-needle array.
Fig. 10 is a view for describing a step of puncturing the skin with the micro-needle array.
Fig. 11 is a view for describing a step of puncturing the skin with the micro-needle array.
Fig. 12 is a cross-sectional view illustrating a micro-needle array unit in another form.
Fig. 13 is a plan view of the micro-needle array unit illustrated in Fig. 12.
Fig. 14 is a cross-sectional view illustrating a micro-needle array unit in still another form.
Fig. 15 is a plan view of the micro-needle array unit illustrated in Fig. 14.
Fig. 16 is a graph showing experimental results of air permeability.

### DESCRIPTION OF THE PREFERRED EMBODIMENTS

Hereinafter, a micro-needle array unit and a container according to the embodiment of the present invention will be described with reference to the accompanying drawings.

The micro-needle array unit according to the present embodiment includes a micro-needle array and a container that accommodates the micro-needle array. Further, the container comprises an accommodating portion that accommodates the micro-needle array, and a lid member that seals an opening provided in the accommodating portion. At least a part of the lid member is formed of a transparent film. Further, a part of the container comprises a water vapor permeable antibacterial film. Hereinafter, preferred embodiments will be described.

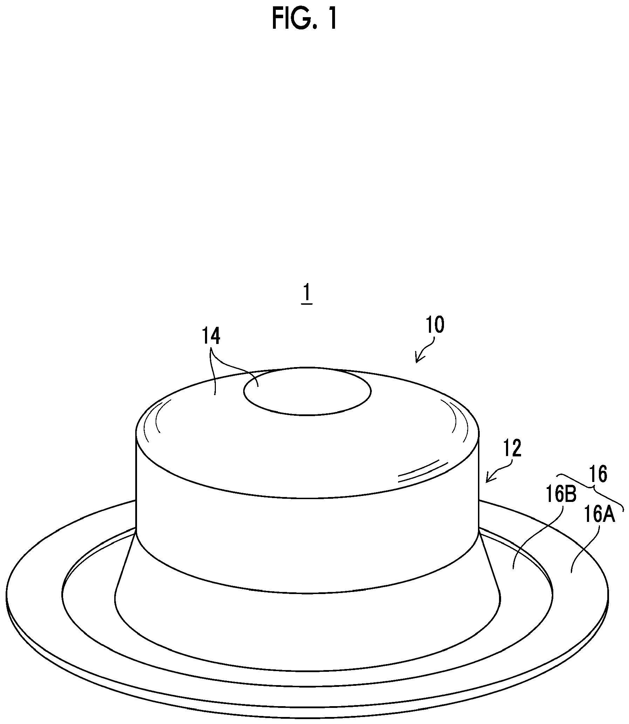

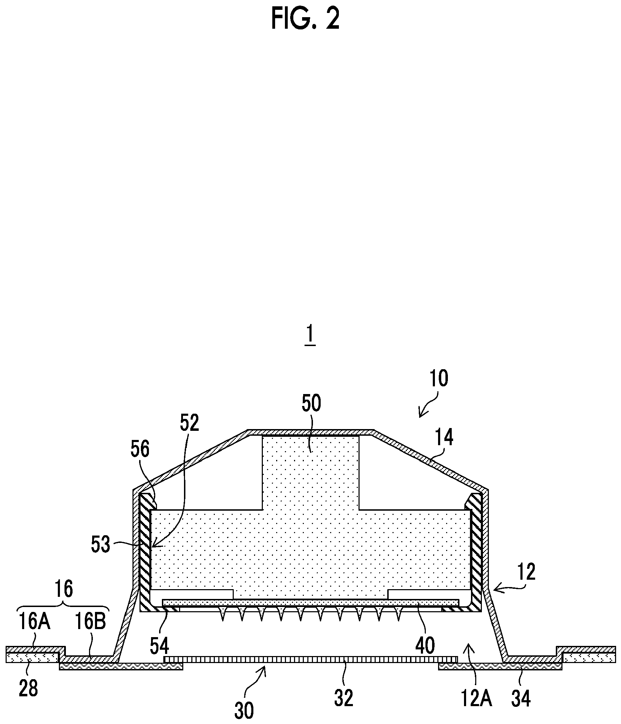

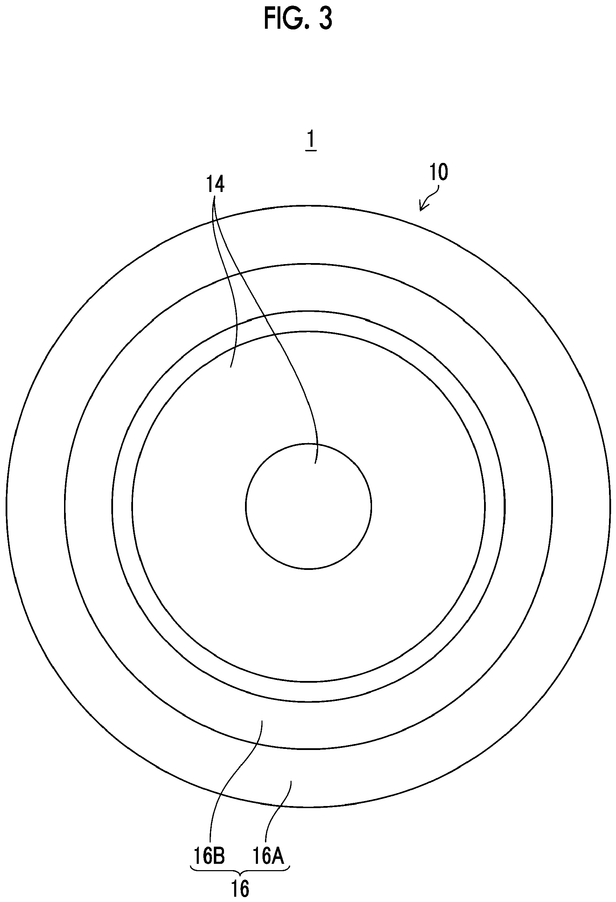

Fig. 1 is a perspective view illustrating a micro-needle array unit. Fig. 2 is a cross-sectional view of the micro-needle array unit illustrated in Fig. 1. Fig. 3 is a cross-sectional view of the micro-needle array unit illustrated in Fig. 1.

As illustrated in Figs. 1 and 2, a micro-needle array unit 1 comprises a micro-needle array 40 and a container 10 that accommodates the micro-needle array 40. The container 10 comprises an accommodating portion 12 that has an opening 12A and accommodates the micro-needle array 40, a deformable portion 14 that is formed integrally with the accommodating portion 12, and a flange portion 16 that is formed integrally with the accommodating portion 12 and extends from the periphery of the opening 12A toward the outside.

The accommodating portion 12, the deformable portion 14, and the flange portion 16 of the container 10 have a circular shape in plan view as illustrated in Fig. 3. However, the shapes of the accommodating portion 12, the deformable portion 14, and the flange portion 16 are not limited thereto. The flange portion 16 is formed of an outer flange portion 16A that is provided on the outside of the flange portion 16 and comes into contact with the skin in a case of puncture of the skin with the micro-needle array 40 and an inner flange portion 16B that is provided inside the outer flange portion 16A and provided in close contact with the lid member 30. According to the embodiment illustrated in Figs. 1 to 3, the flange portion 16 is provided over the entire periphery of the accommodating portion 12. The entire periphery indicates that the entire periphery of the accommodating portion 12 is enclosed by the flange portion 16. Further, in the flange portion 16, since the inner flange portion is in close contact with the lid member 30, it is preferable that the inner flange portion 16B is provided over the entire periphery of the accommodating portion 12, but the outer flange portion 16A is not necessarily provided over the entire periphery of the accommodating portion 12.

It is preferable that the outer flange portion 16A has an adhesive 28 on the surface that is brought into contact with the skin (the surface of the outer flange portion on a side of the same direction as the side of the one surface of the micro-needle array 40). The container 10 is attached to the skin with the adhesive 28 of the outer flange portion 16A. Even in a case where the outer flange portion 16A does not have an adhesive, the container 10 is attached to the skin with an adhesive applied to the skin. Further, the container 10 is attached to the skin by attaching another member (medical tape) or the like from above the container 10.

As illustrated in Fig. 2, the accommodating portion 12 has an internal space defined by an inner wall and the opening 12A. The opening 12A of the accommodating portion 12 is sealed by the lid member 30. The lid member 30 is sealed by adhesion of the periphery of the lid member 30 to the inner flange portion 16B. The accommodating portion 12 has a cylindrical shape according to the embodiment, but the shape of the accommodating portion 12 is not limited as long as the micro-needle array can be accommodated therein.

The deformable portion 14 is disposed on a side opposite to the micro-needle array 40 in the accommodating portion 12 with respect to the opening 12A and is formed integrally with the accommodating portion 12. In the embodiment, for example, the deformable portion 14 is formed in a convex shape in a direction of being separated from the micro-needle array 40. The convex shape indicates that the vertex is not positioned in the internal space of the accommodating portion 12. The integration indicates a state where the accommodating portion 12 and the deformable portion 14 are connected with each other. For example, in a case where the accommodating portion 12 is integrated with the deformable portion 14, this integration can be achieved by separately molding the accommodating portion 12 and the deformable portion 14, fitting the accommodating portion 12 and the deformable portion 14 to each other, and welding the accommodating portion and the deformable portion. In a case where the accommodating portion 12 is molded integrally with the deformable portion 14, the integration may be carried out before or after the accommodation of the micro-needle array 40 in the accommodating portion 12. In the case where the accommodating portion 12 is integrated with the deformable portion 14, the integration can be realized by integrally molding the accommodating portion 12 and the deformable portion 14. However, the integration method is not limited to these methods.

The deformable portion 14 may have a frustum shape. In the embodiment, the deformable portion has a truncated cone shape. Further, the deformable portion 14 may have a cone shape such as a conical shape or a pyramid shape or may also have a dome shape. Further, the deformable portion 14 may have, for example, an internal space, and the internal space of the deformable portion 14 can communicate with the internal space of the accommodating portion 12. The accommodating portion 12 has a structure in which the side opposite to the opening 12A is closed by the deformable portion 14.

The flange portion 16 is integrated with the accommodating portion 12 and brought into contact with the skin as described below. In the embodiment, the flange portion 16 extends outward from a position of the opening 12A of the accommodating portion 12. The inner flange portion 16B disposed inside (the opening 12A side) the flange portion 16 is in close contact with the lid member 30 so that the accommodating portion 12 is sealed. The outer flange portion 16A is further disposed outside the inner flange portion 16B. In the embodiment, a step is formed between the outer flange portion 16A and the inner flange portion 16B, but the configuration of the step is not limited thereto. The outer flange portion 16A and the inner flange portion 16B may be formed on the same surface.

The flange portion 16 is formed to be parallel to the sheet of the micro-needle array 40. The concept of parallel includes parallel and substantially parallel. As described below, the shape of the flange portion 16 is not particularly limited as long as the flange portion comes into contact with the skin. In a case where the accommodating portion 12 is integrated with the flange portion 16, the same method as in the case of the integration of the accommodating portion 12 with the deformable portion 14 can be applied.

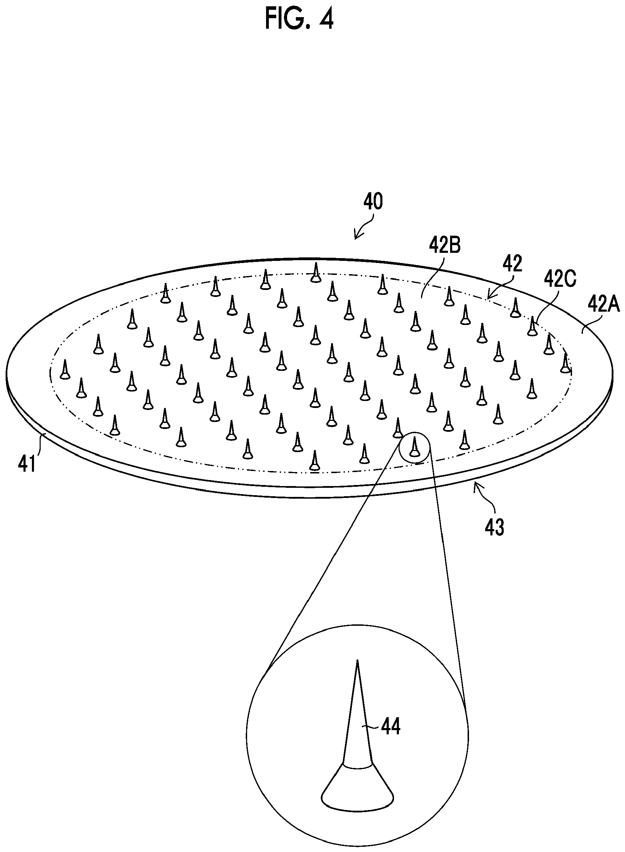

A typical structure of the micro-needle array 40 will be described with reference to Fig. 4. Fig. 4 is a perspective view illustrating the micro-needle array 40. As illustrated in Fig. 4, the micro-needle array 40 comprises a circular sheet 41 having one surface 42 and the other surface 43 which oppose each other and a plurality of needles 44 arranged on the one surface 42 of the sheet 41. The needles 44 constitute micro-needles. The plurality of needles 44 are arranged in a micro-needle region 42B inside an outer peripheral surface 42A of the one surface 42. As illustrated in Fig. 4, the boundary between the outer peripheral surface 42A and the micro-needle region 42B is an imaginary line 42C that connects the needles 44, which are arranged on the outermost side of the micro-needle region 42B, from among the plurality of needles 44. According to the embodiment, an example in which the sheet 41 has a circular shape has been described, but the sheet 41 may have a rectangular shape.

The shape and the size of the sheet 41 and the needles 44 may be selected according to the applications of the micro-needle array 40. Further, the sheet 41 and the needles 44 may be formed of the same material or different materials. The micro-needle array 40 can be produced by integrally molding the sheet 41 and the needles 44, but the sheet 41 and the needles 44 may be molded separately.

The needles 44 respectively have, for example, a substantially cone shape, but may have a columnar shape or a frustum shape. According to the embodiment, the needles 44 are formed in order of a truncated cone portion and a cone from the one surface 42 toward the tip, but the shape thereof is not particularly limited as long as the skin can be punctured by the needles. It is preferable that the needles 44 are arranged in an array in a state of columns (lateral rows) and rows (vertical rows) at equal intervals.

It is preferable that each needle 44 is formed of a material that is dissolved after the puncture into the skin or the mucous membrane and after the insertion into the body. Accordingly, as the material constituting the needles 44, a water-soluble polymer is preferable, and polysaccharides are more preferable. As the material constituting the needles 44, it is preferable that the needles are formed of at least one material selected from the group consisting of hydroxy ethyl starch, dextran, chondroitin sulfate, sodium hyaluronate, carboxymethyl cellulose, polyvinylpyrrolidone, polyoxyethylene polyoxypropylene glycol, and polyethylene glycol.

The sheet 41 of the micro-needle array 40 has a diameter of 10 mm or greater and 30 mm or less and a thickness of 0.1 mm or greater and 5 mm or less. Further, each needle 44 has a length of 0.2 mm or greater and 1.5 mm or less. Further, the number of needles 44 to be arranged on the one surface 42 of the sheet 41 is 4 or greater and 1000 or less. However, the values are not limited thereto.

Returning to Fig. 2, the micro-needle array 40 is installed in the accommodating portion 12 by the resin block 50 and the fixing member 52 provided inside the accommodating portion 12 of the container 10. The resin block 50 is formed in a convex shape and is disposed in the accommodating portion 12 such that the convex portion is disposed on the deformable portion 14 side. The resin block 50 is connected to the container 10 by fitting the convex portion (not illustrated) provided in the deformable portion 14 of the container 10 and a concave portion (not illustrated) formed in the resin block 50 to each other.

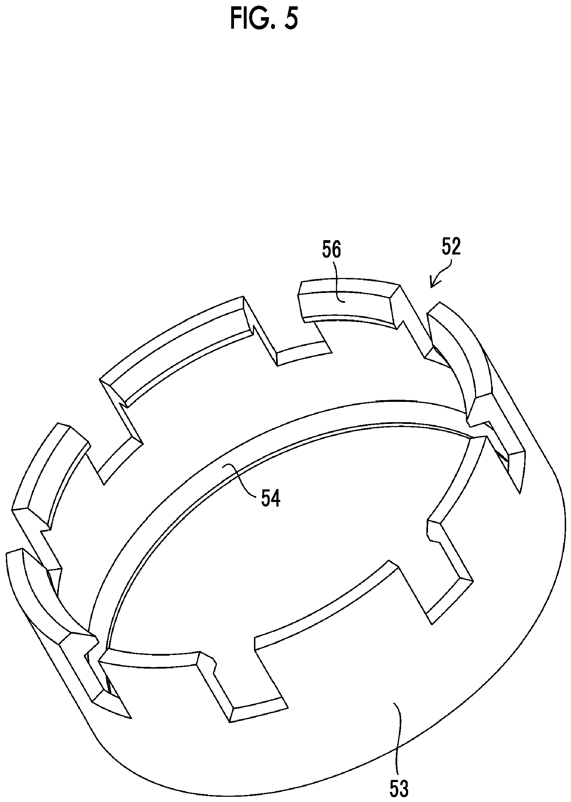

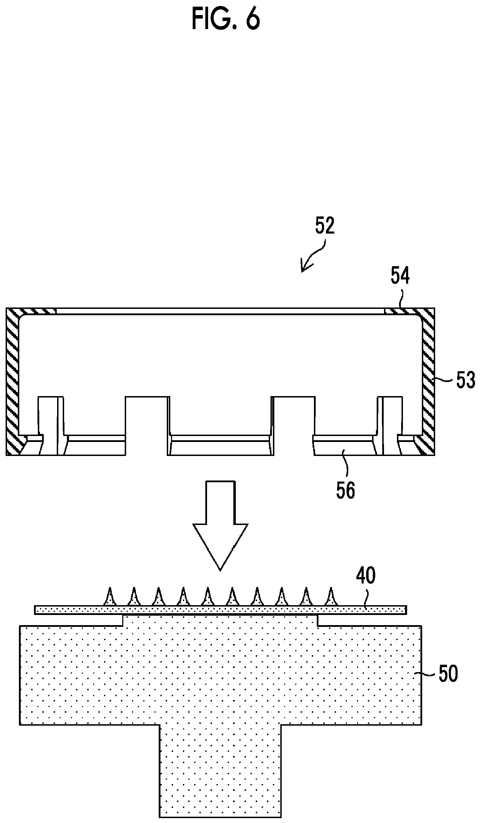

Figs. 5 and 6 are views for describing a method of fixing the micro-needle array to the resin block. Fig. 5 is a perspective view illustrating the fixing member, and Fig. 6 is a view for describing a state in which the fixing member is fitted to the resin block.

The fixing member 52 includes a main body portion 53 formed in a tubular shape, and claw portions 54 and 56 that are fixed in the vertical direction of the resin block 50 inside the end portions on both sides of the main body portion 53. In Fig. 5, the claw portion 54 is formed over the entire periphery of one end portion of the fixing member 52, but the claw portion 56 is formed on the other side at an interval. The shapes of the fixing member 52 and the claw portions 54 and 56 can be appropriately changed depending on the shapes of the resin block 50 and the micro-needle array 40. Further, it is preferable that the claw portion 54 is fixed to be provided over the entire periphery of the fixing member 52 on the side where the micro-needle array 40 is supported. By providing the claw portion 54 over the entire periphery of the fixing member 52 and fixing the micro-needle array 40, it is possible to prevent the micro-needle array 40 from falling off from the resin block 50.

As illustrated in Fig. 6, the micro-needle array 40 is installed on the resin block 50 in a case of fixing the micro-needle array 40 to the resin block 50. Further, by fitting the fixing member 52 from the side where the micro-needle array 40 is installed, the fixing member 52 is fixed to the resin block 50, and the outer peripheral surface 42A of the micro-needle array 40 is supported by the claw portion 54. By connecting the resin block 50 to the deformable portion 14 of the container 10 in a state in which the micro-needle array 40 is fixed to the resin block 50, the micro-needle array 40 is accommodated in the internal space of the accommodating portion 12 in a state in which the needles 44 of the micro-needle array 40 are directed toward the opening 12A.

The resin block 50 and the fixing member 52 are accommodated in the accommodating portion 12 such that the outer periphery of the fixing member 52 and the inner periphery of the accommodating portion 12 are substantially parallel to each other. In this manner, the pushed resin block 50 can be pushed straight out in the direction of the opening 12A by pressing the deformable portion 14.

As described above, the micro-needle array 40 is supported by the claw portion 54 of the fixing member 52 fixed to the resin block 50. Further, the resin block 50 is fixed by being fitted to the convex portion provided on the deformable portion 14 of the container 10. Therefore, the container 10 and the micro-needle array 40 are configured such that the micro-needle array 40 is disposed in the container 10 without using an adhesive in a state in which the needles 44 of the micro-needle array 40 are directed to the opening 12A. In this manner, it is possible to prevent the needles 44 from being damaged due to contact in the container 10. Further, since foreign matter or bacteria attached to the adhesive are difficult to remove, a risk of the foreign matter or bacteria being mixed into the container 10 is increased in a case of using the adhesive in the container, but the risk can be eliminated by not using the adhesive.

Returning to Fig. 2, the container 10 allows the opening 12A to be sealed with the lid member 30. The lid member 30 can be bonded by heat sealing. At least a part of the lid member 30 to be used is formed of a transparent film 32. By forming a part of the lid member 30 with the transparent film 32, the micro-needle array 40 in the accommodating portion 12 can be visually recognized without peeling off the lid member 30. As the transparent film, a polyethylene resin, a polypropylene resin, or the like can be used. Further, the term "transparency" indicates that the transmittance of all visible light beams is 85% or greater.

Further, in the embodiment, a part of the lid member 30 is formed of a water vapor permeable antibacterial film 34. The water vapor permeable antibacterial film may be provided over the entire periphery of the transparent film 32 or may be provided on a part of the lid member 30. The water vapor permeable antibacterial film 34 indicates a film that allows water vapor to permeate therethrough, but suppresses the invasion of bacteria. In order to suppress the invasion of bacteria, the pore diameter of the water vapor permeable antibacterial film is set to preferably 0.2 µm or less. As the water vapor permeable antibacterial film 34, for example, high-density polyethylene non-woven fabric can be used. As the high-density polyethylene non-woven fabric, "TYVEK" (manufactured by DuPont de Nemours, Inc.) can be used.

By forming a part of the lid member 30 with the water vapor permeable antibacterial film 34, water vapor can be allowed to permeate through the film even after the opening 12A is sealed with the lid member 30, and thus the micro-needle array 40 can be dried. Further, the invasion of bacteria into the accommodating portion 12 of the container 10 can be prevented. Therefore, the micro-needle array 40 can be dried even after being accommodated in the container 10 while the sterile state is maintained. In this manner, since the micro-needle array 40 is not necessarily dried in a sterile room and can be dried regardless of the drying place, it is possible to prevent the drying from taking a long time in a step of producing the micro-needle array 40.

In order to dry the micro-needle array 40 after sealing the container with the lid member 30, the area of the water vapor permeable antibacterial film 34 is set to preferably 18 mm² or greater and 250 mm² or less and more preferably 30 mm² or greater and 137 mm² or less. By setting the area of the water vapor permeable antibacterial film 34 to be in the above-described range, the micro-needle array 40 can be dried. Further, in the present embodiment, since the water vapor permeable antibacterial film 34 is provided on the lid member 30, it is preferable that the area of the water vapor permeable antibacterial film 34 provided on the lid member 30 is set to be in the above-described range. However, in a case where the water vapor permeable antibacterial film is provided on the deformable portion or the flange portion as in another embodiment described below, it is preferable that the area of the entire container is set to be in the above-described range.

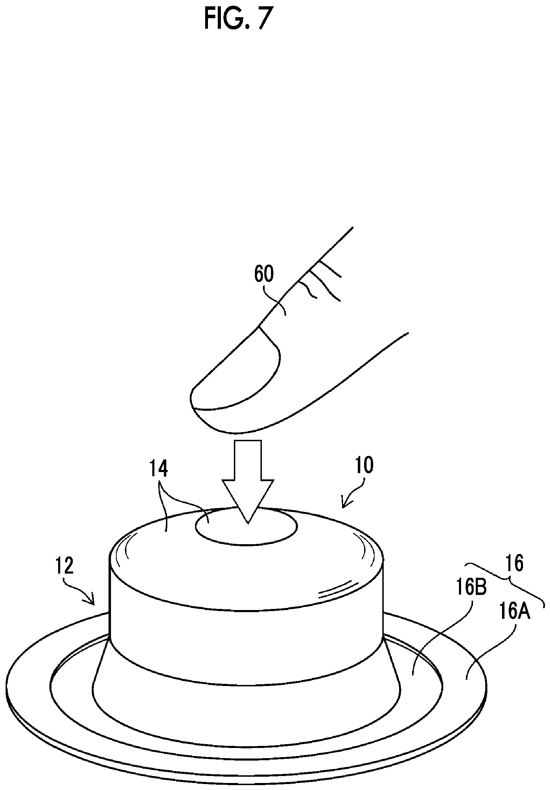

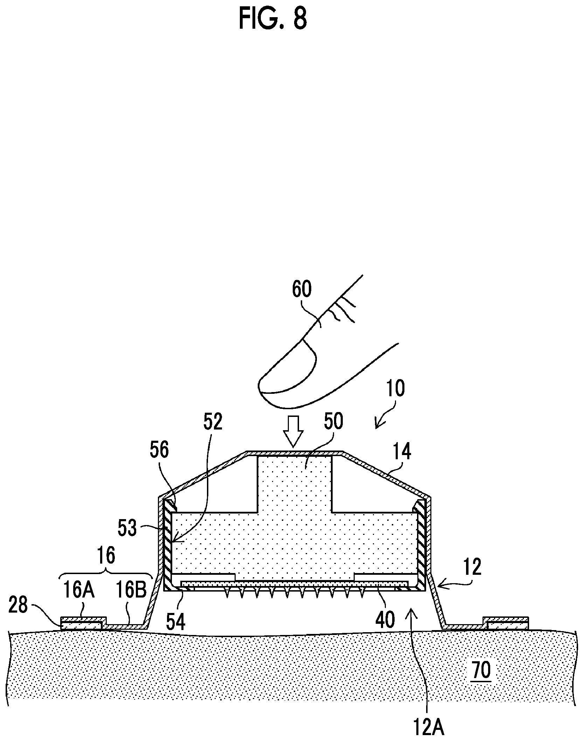

Next, a step of puncturing the skin with the micro-needle array 40 using the micro-needle array unit 1 will be described with reference to Figs. 7 to 11. The configurations which are the same as the configurations described in Figs. 1 to 3 are denoted by the same reference numerals, and the description thereof will not be provided. Fig. 7 is a perspective view of the micro-needle array unit illustrating the step of puncturing the skin with the micro-needle array 40. Figs. 8 to 11 are cross-sectional views of the micro-needle array unit 1 illustrating the step of puncturing the skin with the micro-needle array 40.

First, the lid member 30 that seals the opening 12A of the accommodating portion 12 is peeled off from the container 10. The needles 44 of the micro-needle array 40 are protected from damage because of the lid member 30. It is preferable that the lid member 30 has a knob in order to facilitate the peeling.

Next, the container 10 is positioned on the skin as illustrated in Fig 7. The opening 12A of the accommodating portion 12 is positioned toward the skin so that the needles 44 (not illustrated) of the micro-needle array 40 are directed to the skin. An external force in a direction of the opening 12A is applied to the deformable portion 14 using a finger 60.

Fig. 8 is a cross-sectional view of Fig. 7. As illustrated in Fig. 8, the container 10 is positioned on the skin 70. The flange portion 16 extending outward from the accommodating portion 12 is brought into contact with the skin 70. The finger is positioned at a position separated from the deformable portion 14 in order to apply an external force to the deformable portion 14 in the direction of the opening 12A. The micro-needle array 40 is supported by the claw portion 54 of the fixing member 52 and positioned in the internal space of the accommodating portion 12.

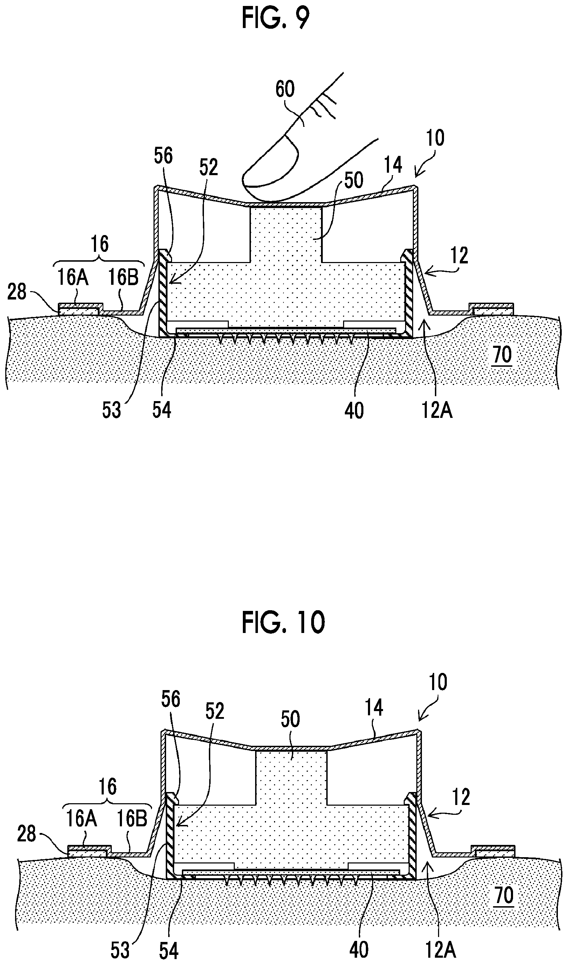

As illustrated in Fig. 9, the deformable portion 14 is pressed toward the skin 70 using the finger 60. The deformable portion 14 is deformed by receiving the external force in the direction of the opening 12A. As described above, since the resin block 50 is fixed to the container 10 by fitting the convex portion provided on the deformable portion 14 and the concave portion provided on the resin block 50 to each other, the other surface 43 of the micro-needle array 40 is pressed by pressing the deformable portion 14 through the resin block 50. The micro-needle array 40 is pushed out of the accommodating portion 12 by pressing the other surface 43 in a state in which the micro-needle array 40 is supported by the claw portion 54 of the fixing member 52. The micro-needle array 40 passes through the opening 12A so that the needles 44 of the micro-needle array 40 puncture the skin 70.

As illustrated in Fig. 10, the deformable portion 14 is deformed by the external force. Even after the external force is removed, the deformable portion 14 maintains the deformed shape. The deformed deformable portion 14 presses the micro-needle array 40 toward the skin 70.

After the puncture, since the micro-needle array 40 is pressed by the deformable portion 14 of the container 10 until the drug of the micro-needle array 40 is administered, falling of the micro-needle array 40 off the skin 70 without pressing of the finger 60 is prevented.

In a case where the deformable portion 14 is pressed, the resin block 50 disposed in the accommodating portion 12 is pressed. In the embodiment, the resin block 50 has a convex shape, and the thus the area on the side where the micro-needle array 40 is disposed is larger than the area on the side of the deformable portion 14. In this manner, a force can be uniformly applied to the surface of the resin block 50 on a side opposite to the deformable portion 14 by pressing the deformable portion 14 so that the needles 44 of the micro-needle array 40 can uniformly puncture the skin 70.

Further, by designing the outer diameter in a case where the fixing member 52 is mounted on the resin block 50 to be slightly smaller than the inner diameter of the accommodating portion 12, a large deviation of the pressed resin block 50 from the direction of the opening 12A can be prevented. Therefore, the skin 70 can be vertically punctured by the needles 44 of the micro-needle array 40.

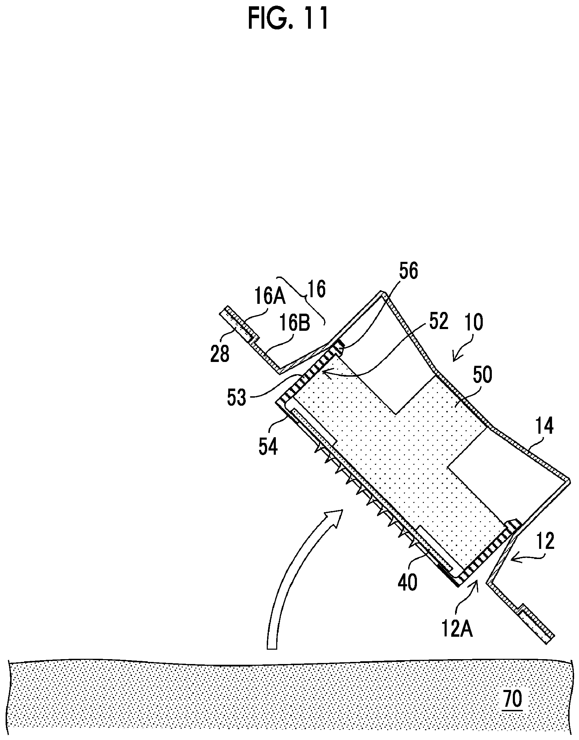

Finally, the micro-needle array 40 and the container 10 are peeled off as illustrated in Fig 11. The peeling is performed after the time until the skin 70 is punctured by the needles 44 of the micro-needle array 40 and the drug contained in the needles 44 is administered into the skin has elapsed. In this manner, the drug can be injected into the skin.

It is preferable that the container 10 constituting the micro-needle array unit is formed of, for example, a polyethylene resin, a polypropylene resin, or a mixture thereof. However, the materials are not limited thereto. It is preferable that these materials respectively satisfy the "Specification of Plastic Container for Aqueous Injections (hereinafter, simply referred to as an injection container grade) of Japanese Pharmacopoeia". In addition, the container 10 may be formed of various other resin materials satisfying the same specification.

In particular, a material in which the shape is deformed in a case of the deformable portion 14 receiving an external force and the deformed shape is maintained is selected from among these materials. The material to be used is determined in consideration of the shape and the thickness of the deformable portion 14 and the magnitude of the external force required for the deformation.

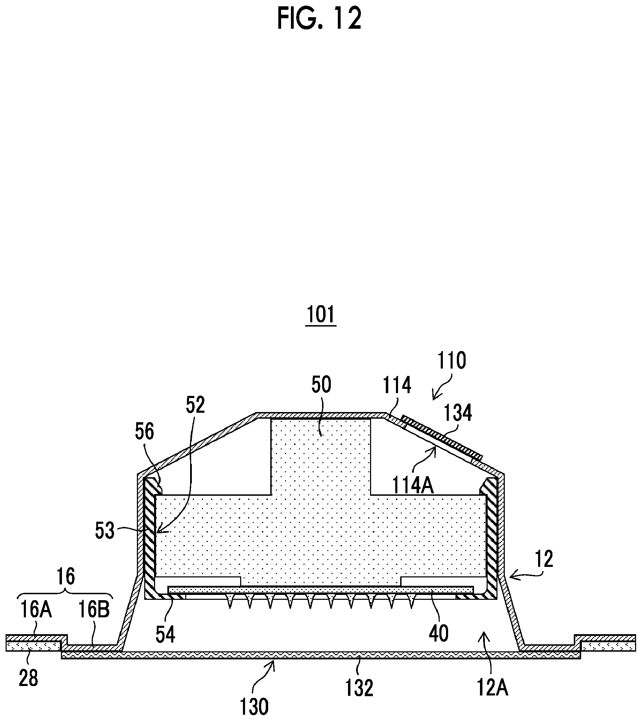

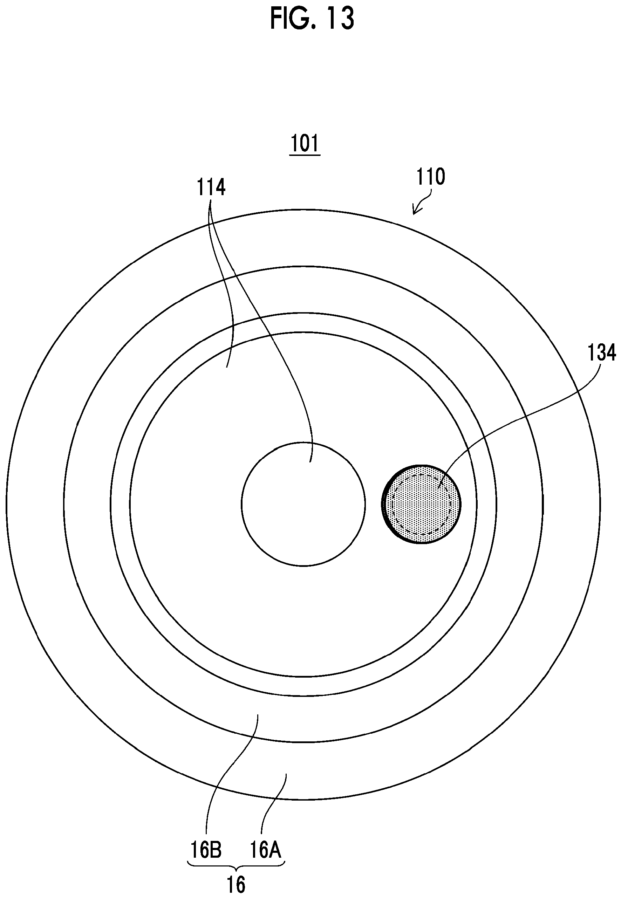

Fig. 12 is a cross-sectional view illustrating a micro-needle array unit 101 in another form, and Fig. 13 is a plan view of the micro-needle array unit 101. The configurations which are the same as those of the micro-needle array unit 1 illustrated in Fig. 1 are denoted by the same reference numerals, and the description thereof will not be provided.

The micro-needle array unit 101 illustrated in Fig. 12 is different from the micro-needle array unit 1 illustrated in Fig. 1 in terms of the position where a water vapor permeable antibacterial film 134 is provided in a container 110. The micro-needle array unit 101 of Figs. 12 and 13 is different from the micro-needle array unit 1 in terms that the water vapor permeable antibacterial film 134 is provided on an opening 114A formed in the deformable portion 114. In this case, the lid member 130 may be formed of only a transparent film 132, or a part of the lid member 130 may be formed of a transparent film.

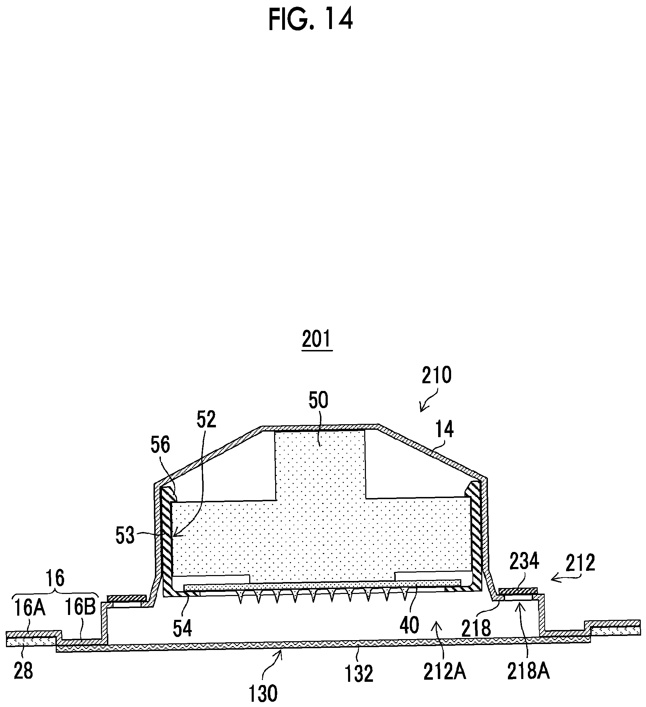

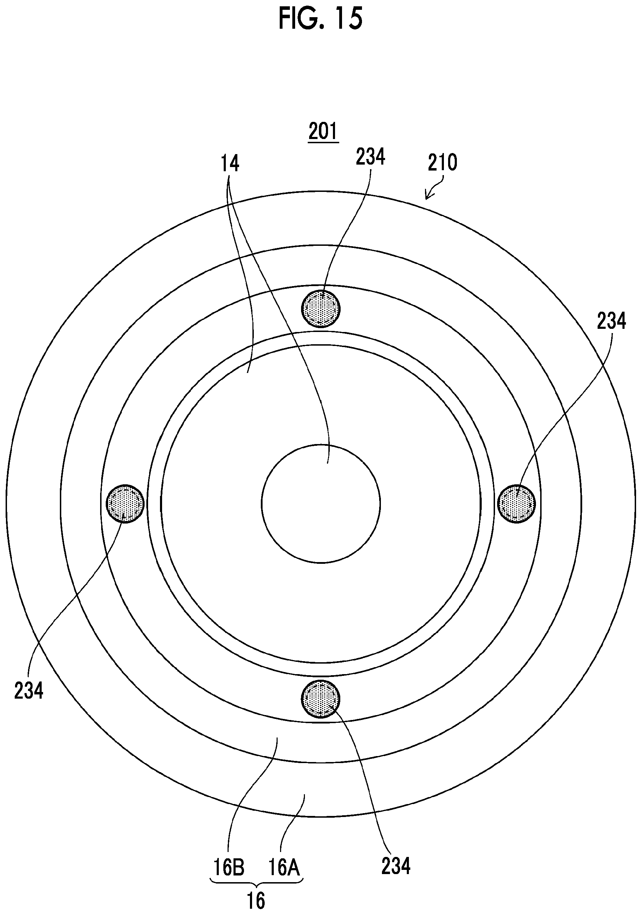

Further, Fig. 14 is a cross-sectional view illustrating a micro-needle array unit 201 in still another form, and Fig. 15 is a plan view of the micro-needle array unit 201. The micro-needle array unit illustrated in Fig. 14 is different from the micro-needle array unit 1 illustrated in Fig. 1 in terms that a stepped portion 218 is provided in an accommodating portion 212 inside an inner flange portion 16B of a container 210 and a water vapor permeable antibacterial film 234 is provided on an opening 218A formed in the stepped portion 218. The opening 212A of the accommodating portion 212 is sealed with the lid member 130 consisting of the transparent film 132. Similar to the micro-needle array unit illustrated in Fig. 12, the lid member 130 may be formed of only the transparent film 132 or a part of the lid member 130 may be formed of the transparent film.

Similar to the micro-needle array unit illustrated in Figs. 12 to 15, by providing the water vapor permeable antibacterial films 134 and 234 on a part of the containers 110 and 210, the water vapor generated by drying the micro-needle array can be discharged from the water vapor permeable antibacterial film 134 and 234 even after the accommodating portions 12 and 212 are sealed with the lid member 130. Further, the invasion of bacteria into the accommodating portions 12 and 212 can be prevented. In this manner, even in a case where the micro-needle array has not been completely dried, the micro-needle array can be dried after packaging of the micro-needle array, and the invasion of bacteria can be prevented.

Therefore, the micro-needle array can be dried even in a case where a sterile state (in a sterile room) is not prepared.

Next, the effects of the sterility, the air permeability, and the internal visibility of the container will be described based on experiments.

### «Sterility»

The sterility was tested with reference to the antibacterial test described in the guidelines for packaging sterilized medical devices. In the container 10, a container containing an agar medium in place of the micro-needle array and a dry fine powder soil containing bacteria were placed in a decompression desiccator. In addition, a control sample was prepared by punching a hole in the container 10 and placing a container containing an agar medium in the container 10. Next, the dry fine powder soil in the desiccator was diffused by reducing the pressure in the desiccator and rapidly returning the pressure to the atmospheric pressure. The container was taken out from the desiccator, and the agar medium was cultured at 37°C for 48 hours. Visual confirmation whether visible colonies were formed in the agar medium after cultivation was performed.

Visible colonies were not found in the agar medium cultured using the container of the present embodiment, but visible colonies were found in the control sample. Based on the result, it can be confirmed that the container of the present embodiment can be maintained in a sterile state without allowing bacteria to pass through the accommodating portion.

### «Air permeability»

The water content of the micro-needle array 40 was measured by placing the micro-needle array 40 in the container 10 of the present embodiment and drying the micro-needle array 40 in an environment of -40°C DP (Dew Point). As a control sample, the micro-needle array 40 without packaging and a container of the related art in which only a transparent film was used as the lid member were prepared and the micro-needle array was dried in the same manner as described above. Further, in a case where the micro-needle array was dried in the container, a plurality of samples were prepared, each container was opened after a predetermined period of time had elapsed, and the water content of each micro-needle array was measured. One day after the measurement of the present embodiment, two samples were measured.

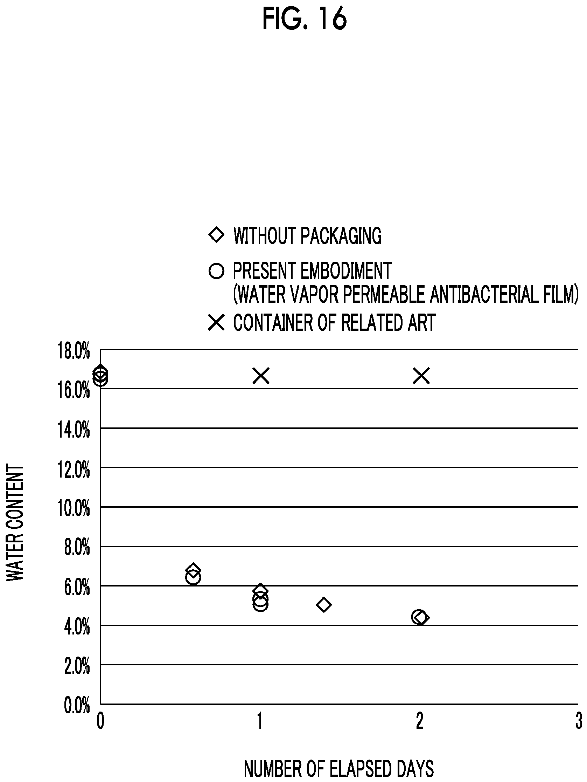

The results are shown in Fig. 16. It was confirmed that the water content of the micro-needle array 40 dried in the container 10 of the present embodiment was decreased as in the case of the micro-needle array 40 without packaging and thus the micro-needle array 40 was able to be dried in a state of being packaged in the container 10.

### «Internal visibility»

Since a transparent film is used as the lid member in the container of the present embodiment, the inside the container can be visually recognized.

As described above, according to the present embodiment, the invasion of bacteria into the container can be prevented and the sterile state can be maintained by forming a part of the container with a water vapor permeable antibacterial film. Further, since water vapor can be allowed to permeate through the container, the micro-needle array can be dried after being packaged in the container. In this manner, a delay of the drying step due to the reason that a sterile room cannot be used can be prevented. Further, since a transparent film is used as the lid member, visibility inside the container can be ensured.

### Explanation of References

1, 101, 201: micro-needle array unit
10, 110, 210: container
12, 212: accommodating portion
12A, 114A, 212A, 218A: opening
14, 114: deformable portion
16: flange portion
16A: outer flange portion
16B: inner flange portion
28: adhesive
30, 130: lid member
32, 132: transparent film
34, 134: water vapor permeable antibacterial film
40: micro-needle array
41: sheet
42: one surface
42A: outer peripheral surface
42B: micro-needle region
42C: imaginary line
43: other surface
44: needle
50: resin block
52: fixing member
53: main body portion
54, 56: claw portion
60: finger
70: skin
218: stepped portion

## Claims

1. A micro-needle array unit comprising:
a micro-needle array which includes a sheet and a plurality of needles arranged inside an outer peripheral surface of one surface of the sheet; and
a container which accommodates the micro-needle array,
wherein the container includes
an accommodating portion having an opening,
a claw portion provided in the accommodating portion and supporting the outer peripheral surface of the one surface of the micro-needle array,
a deformable portion disposed on a side opposite to the opening and formed integrally with the accommodating portion,
a flange portion extending from a periphery of the opening and formed integrally with the accommodating portion, the flange portion consisting of an outer flange portion that comes into contact with a skin in a case where the skin is punctured by the micro-needle array and an inner flange portion that is provided inside the outer flange portion, and
a lid member provided in close contact with the inner flange portion,
at least a part of the lid member is formed of a transparent film,
a water vapor permeable antibacterial film is provided on at least a part of the container,
the deformable portion is deformed by receiving an external force in a direction of the opening and presses the other surface of the micro-needle array, and
the micro-needle array is pushed out of the accommodating portion by pressing the other surface, and the deformable portion maintains a deformed state and presses the micro-needle array.

2. The micro-needle array unit according to claim 1,
wherein the micro-needle array is formed of a water-soluble polymer.

3. The micro-needle array unit according to claim 1 or 2,
wherein an area of the water vapor permeable antibacterial film is 18 mm² or greater and 250 mm² or less.

4. The micro-needle array unit according to any one of claims 1 to 3,
wherein the deformable portion has a convex shape separated from the micro-needle array.

5. The micro-needle array unit according to claim 4,
wherein the convex shape is a dome shape or a truncated cone shape.

6. The micro-needle array unit according to any one of claims 1 to 5,
wherein the outer flange portion has an adhesive on a side of the same direction as a side of the one surface of the micro-needle array.

7. The micro-needle array unit according to any one of claims 1 to 6,
wherein the flange portion is provided over an entire periphery of the accommodating portion.

8. The micro-needle array unit according to any one of claims 1 to 7, further comprising:
a resin block which is provided in the accommodating portion and disposed in connection with the deformable portion; and
a fixing member which has the claw portion and is fixed by being fitted to a periphery of the resin block,
wherein the micro-needle array is supported by the fixing member having the claw portion.

9. The micro-needle array unit according to any one of claims 1 to 8, wherein the water vapor permeable antibacterial film is provided on the lid member.

10. The micro-needle array unit according to any one of claims 1 to 8, wherein the water vapor permeable antibacterial film is provided on the deformable portion.

11. The micro-needle array unit according to any one of claims 1 to 8, wherein the water vapor permeable antibacterial film is provided on the accommodating portion.

12. A container which accommodates a micro-needle array having a sheet and a plurality of needles arranged inside an outer peripheral surface of one surface of the sheet, the container comprising:
an accommodating portion which has an opening;
a claw portion which is provided in the accommodating portion and supports the outer peripheral surface of the one surface of the micro-needle array;
a deformable portion which is disposed on a side opposite to the opening and formed integrally with the accommodating portion;
a flange portion which extends from a periphery of the opening and is formed integrally with the accommodating portion, the flange portion consisting of an outer flange portion that comes into contact with a skin and an inner flange portion that is provided inside the outer flange portion; and
a lid member which is provided in close contact with the inner flange portion,
wherein at least a part of the lid member is formed of a transparent film,
a water vapor permeable antibacterial film is provided on at least a part of the container,
the deformable portion is deformed by receiving an external force in a direction of the opening and presses the other surface of the micro-needle array, and
the deformable portion maintains a deformed state and presses the micro-needle array.
